# FASCICULE DE BREVET EUROPEEN

(11) **EP 2 079 316 B1**
(45) Date de publication et mention de la délivrance du brevet: **05.11.2014**
(21) Numéro de dépôt: 07848298.1
(22) Date de dépôt: 01.10.2007
(51) Int. Cl.: A23C 9/12, A23L 1/053, A23L 3/3571, C12N 1/20

(54) **UTILISATION DE LA GOMME ARABIQUE POUR AMELIORER LA CROISSANCE ET LA SURVIE DES BIFIDOBACTERIES.**
VERWENDUNG VON GUMMI ARABIKUM ZUR VERBESSERUNG DES WACHSTUMS UND DER ÜBERLEBENSFÄHIGKEIT DER BIFIDOBAKTERIEN
USE OF GUM ARABIC FOR IMPROVING THE GROWTH AND SURVIVAL OF BIFIDOBACTERIA

(30) Priorité: 02.10.2006 FR 0608613
(43) Date de publication de la demande: 22.07.2009
(73) Titulaire: Compagnie Gervais Danone, 75009 Paris (FR)
(72) Inventeur: TERRAGNO, Luc, F-92190 Meudon (FR); DEBRU, François, F-78000 Versailles (FR); HERVE, Stéphane, F-92330 Sceaux (FR); TEISSIER, Philippe, F-91120 Palaiseau (FR)
(74) Mandataire: Rançon, Xavier Lucien Abel
(86) Numéro de dépôt international: PCT/FR2007/001598
(87) Numéro de publication internationale: WO 2008/040872

(56) Documents cités:
- WO-A-99/04649
- FR-A1- 2 815 823
- US-A- 5 952 021
- US-A1- 2005 233 049
- US-A1- 2006 057 131
- CROCIANI F ET AL: "DEGRADATION OF COMPLEX CARBOHYDRATES BY BIFIDOBACTERIUM SPP" INTERNATIONAL JOURNAL OF FOOD MICROBIOLOGY, ELSEVIER SCIENCE PUBLISHERS, AMSTERDAM, NL, vol. 24, 1994, pages 199-210, XP001009939 ISSN: 0168-1605
- WEN-CHIAN LIAN ET AL: "Survival of bifidobacteria after spray-drying." INTERNATIONAL JOURNAL OF FOOD MICROBIOLOGY, vol. 74, no. 1/2, 2002, pages 79-86, XP009085030 ISSN: 0168-1605 cité dans la demande
- LIAN WEN-CHIAN ET AL: "Viability of microencapsulated bifidobacteria in simulated gastric juice and bile solution." INTERNATIONAL JOURNAL OF FOOD MICROBIOLOGY, [Online] vol. 86, no. 3, 15 septembre 2003 (2003-09-15), pages 293-301, XP002437472 ISSN: 0168-1605 Extrait de l'Internet: URL:http://www.sciencedirect.com/science?_ ob=MImg&_imagekey=B6T7K-482YWCK-1-9&_cdi=5 061&_user=987766&_orig=search&_coverDate=0 9%2F15%2F2003&_sk=999139996&view=c&wchp=dG LzVlz-zSkWW&md5=b5f9793c192fbb61b7bf30bc78 3b37f0&ie=/sdarticle.pdf> [extrait le 2007-06-11] cité dans la demande
- HSIAO HUNG-CHI ET AL: "Effect of packaging conditions and temperature on viability of microencapsulated bifidobacteria during storage." JOURNAL OF THE SCIENCE OF FOOD AND AGRICULTURE, [Online] vol. 84, no. 2, 5 janvier 2004 (2004-01-05), pages 134-139, XP002437473 ISSN: 0022-5142 Extrait de l'Internet: URL:http://www3.interscience.wiley.com/cgi -bin/fulltext/106599437/PDFSTART> [extrait le 2007-06-11] cité dans la demande
- MICHEL C ET AL: "In vitro prebiotic effects of Acacia gums onto the human intestinal microbiota depends on both botanical origin and environmental pH." ANAEROBE, [Online] vol. 4, no. 6, décembre 1998 (1998-12), pages 257-266, XP002437632 ISSN: 1075-9964 Extrait de l'Internet: URL:http://www.sciencedirect.com/science?_ ob=MImg&_imagekey=B6W9T-45KKV5M-2-1&_cdi=6 691&_user=987766&_orig=browse&_coverDate=1 2%2F31%2F1998&_sk=999959993&view=c&wchp=dG LbVzz-zSkWA&md5=729d3ae7d174b3a66437e1fed4 f836d1&ie=/sdarticle.pdf> [extrait le 2007-06-14]

## Description

L'invention est relative à l'utilisation de la gomme d'acacia (ou gomme arabique), seule ou en association avec un acide aminé soufré, pour améliorer la croissance et la survie de bifidobactéries entrant dans la fabrication de produits alimentaires fermentés.

Les bifidobactéries font partie des microorganismes probiotiques les plus largement utilisés à l'heure actuelle pour la fabrication de différents produits alimentaires, et notamment des produits laitiers, fermentés.

On définit comme probiotiques des "microorganismes vivants, qui lorsqu'ils sont consommés en quantités adéquates, ont un effet bénéfique sur la santé de l'hôte" (Rapport de la consultation mixte d'experts FAO/OMS sur l'évaluation des propriétés sanitaires et nutritionnelles des probiotiques dans les aliments, 2001). Cette définition implique que pour exercer son effet bénéfique, le microorganisme concerné doit être présent en quantité suffisante dans le produit prêt à consommer. Il est généralement considéré que cette quantité doit être au moins égale à 10⁷ ufc/ml. Le maintien de la viabilité des bactéries probiotiques au cours de la fabrication, du conditionnement, et du stockage des produits alimentaires qui les contiennent constitue donc un élément essentiel de la qualité de ces produits.

Les espèces de bifidobactéries les plus employées dans le domaine alimentaire sont *Bifidobacterium adolescentis, B. bifidum, B. breve, B. longum, B. animalis* et *B. infantis.*

Dans le cadre de la fabrication de produits alimentaires fermentés, les bifidobactéries peuvent être utilisées seules ; cependant, elles sont le plus souvent, pour des raisons organoleptiques et/ou technologiques, associées à d'autres bactéries lactiques. Elles sont notamment fréquemment associées à des ferments du yogourt (*L. bulgaricus* et *S. thermophilus*). Elles peuvent être ajoutées aux produits déjà fermentés, ou, plus communément, mélangées aux autres bactéries lactiques, lors de l'ensemencement du substrat à fermenter.

Dans les conditions mises en oeuvre lors de la fabrication de produits alimentaires, la croissance des bifidobactéries est fréquemment lente ; en outre, à l'issue de la fermentation, il est souvent malaisé de maintenir une population de bifidobactéries suffisante, pendant toute la durée de conservation du produit, jusqu'à sa consommation. Ces problèmes peuvent être accentués si les bifidobactéries sont associées avec d'autres bactéries lactiques. Du fait de la faible compétitivité des bifidobactéries en culture mixte, elles ont souvent une croissance plus lente en présence d'autres ferments lactiques que lorsqu'elles sont cultivées seules ; de même, leur survie au cours de la conservation du produit fermenté est généralement moins bonne, notamment du fait que certaines autres bactéries lactiques continuent à produire, entre autres, de l'acide lactique. Ce phénomène, connu sous le nom de post-acidification, affecte la viabilité des bifidobactéries présentes dans le même produit.

Dans ce cadre, il apparaît souhaitable de stimuler la croissance et d'augmenter la viabilité des bifidobactéries, et de préférence, de le faire en n'induisant que peu ou pas d'effets (positifs ou négatifs) sur la croissance et la viabilité des bactéries lactiques auxquelles ces bifidobactéries sont associées.

Les Inventeurs ont découvert que l'utilisation de gomme arabique lors de la fabrication des produits fermentés permettait d'obtenir ces résultats.

La gomme arabique est produite par exudation de blessures des troncs ou des branches de certaines espèces d'acacia. C'est un polysaccharide hydrosoluble de haut poids moléculaire, fortement ramifié. La gomme arabique utilisée en tant qu'additif alimentaire (E414) provient exclusivement d'*Acacia seyal* ou d'*Acacia senegal.*

Il a été proposé d'utiliser la gomme arabique pour encapsuler des bactéries probiotiques (diverses bifidobactéries et *Lactobacillus paracasei*), afin de les préserver de la chaleur et de la déshydratation lors du séchage, et d'améliorer leur viabilité, ainsi que leur résistance aux sucs gastriques et à la bile (Lian et al. 2002 ; Lian et al. 2003 ; Hsiao et al. 2004 ; Desmond et al. 2002).

Il a également été rapporté que l'ingestion de gomme arabique chez l'homme, à raison de 10 grammes par jour, permettait d'augmenter la population de *Bifidobacterium* et de *Bacteroides* dans les selles ; plus récemment, une étude similaire a décrit une augmentation de la population totale de bactéries lactiques, et principalement des *Bifidobacterium* et des *Lactobacillus,* après ingestion de 10 à 15 grammes de gomme arabique par jour (Wyatt et al. 1986 ; Cherbut et al. 2003).

US 5 952 021 A, FR 2 815 823 A et WO 99/04649 décrivent des compositions qui comprennent parmi autres des *Bifidobacteria* et de la gomme arabique.

Les inventeurs ont constaté que lorsque la gomme arabique était additionnée *in vitro* à une culture contenant des bifidobactéries, seules ou avec d'autres bactéries lactiques, elle stimulait la croissance des bifidobactéries au cours de la fermentation, et augmentait leur durée de vie au cours de la conservation du produit fermenté ; en revanche, elle n'avait peu ou pas d'effet sur la croissance et la viabilité des autres bactéries lactiques quand celles-ci étaient présentes. Ils ont de plus constaté que si l'on ajoutait en outre de la cystéine, les effets spécifiques de la gomme arabique sur les bifidobactéries, notamment sur leur survie au cours de la conservation, étaient considérablement augmentés.

La présente invention a pour objet un procédé pour augmenter la viabilité des bifidobactéries au cours de la conservation d'un produit alimentaire frais fermenté contenant une ou plusieurs souche(s) de *Bifidobacterium* caractérisé en ce qu'il comprend l'addition de gomme arabique audit produit alimentaire, préalablement à la fin de son conditionnement.

Selon un mode de mise en oeuvre préféré d'un procédé de la présente invention, ledit produit alimentaire contient en outre une ou plusieurs souche(s) de bactérie(s) lactique(s) autres que *Bifidobacterium.*

Selon un autre mode de mise en oeuvre préféré de la présente invention, ledit procédé comprend en outre l'addition d'au moins un acide aminé soufré (cystéine ou méthionine), de préférence de la cystéine, audit produit alimentaire préalablement à la fin de son conditionnement.

On définit ici par « produit alimentaire fermenté» un produit résultant de la fermentation d'un substrat alimentaire approprié par un ferment comprenant des bactéries lactiques, et dans lequel ces bactéries lactiques demeurent vivantes. Ce produit peut également contenir des bactéries lactiques vivantes qui n'ont pas participé à la fermentation, mais ont été ajoutées à l'issue de celle-ci ; il peut aussi contenir, le cas échéant, des microorganismes utilisables dans la fabrication alimentaire, autres que des bactéries lactiques, par exemple des *Acetobacter* ou des levures. Le terme « frais » indique que ce produit n'a subi, après la fermentation, aucun traitement tel que la lyophilisation ou la dessication. Les produits frais sont généralement conservés par réfrigération.

On définit par « fin du conditionnement », le moment de la fermeture du contenant dans lequel sera conservé le produit fermenté jusqu'à sa consommation.

Les substrats alimentaires appropriés pour la mise en oeuvre de la présente invention sont tous ceux habituellement utilisables pour l'obtention de produits alimentaires fermentés contenant des bifidobactéries.

Il peut s'agir par exemple d'un substrat laitier, qui peut être du lait de vache, de chèvre, de brebis, de jument ou de tout autre mammifère dont le lait est utilisable en alimentation humaine ; ce lait peut éventuellement avoir subi différents traitements permettant de contrôler notamment sa composition en protéines et en matières grasses (écrémage, ultrafiltration, concentration ou dilution, addition de fractions laitières, etc.) ; il peut également s'agir de lait reconstitué à partir de lait en poudre ou de fractions laitières ou de lait enrichi de protéines laitières et/ou d'hydrolysats de protéines laitières.

Il peut s'agir également d'un substrat végétal, par exemple, de jus de fruit ou de légume, de lait de soja, de substrat à base de céréales telles que le blé, l'avoine, le mais, etc.

Il peut s'agir également d'un mélange ou de mélanges de deux ou plus de ces substrats.

Il peut aussi s'agir de substrat tel qu'un fourrage gras.

Le cas échéant ledit substrat alimentaire peut être additionné de différents agents, tels que des agents de texture, de saveur, arômes, etc...

De manière classique, avant l'ensemencement par les bactéries choisies, ledit substrat est traité thermiquement, par exemple à 90-95°C pendant 5 à 10 minutes, afin de détruire les éventuels contaminants bactériens.

La ou les souche(s) de bifidobactéries utilisées pour la mise en oeuvre de la présente invention peuvent être choisies parmi les espèces de bifidobactéries habituellement utilisées pour la préparation de produits alimentaires, mentionnées précédemment. Des souches de bifidobactéries de différentes espèces peuvent être associées. Des espèces préférées sont notamment *Bifidobacterium animalis,* et en particulier les sous-espèces *Bifidobacterium animalis animalis* et *Bifidobacterium animalis lactis.* A titre d'exemple on citera la souche de *Bifidobacterium animalis lactis* CNCM I-2494.

D'autres espèces préférées de bifidobactéries sont *B. lactis* BB12 (CHR Hansen), *B. longum* BB536 (Morinaga), *B. breve* CNCM I- 2219 (Danone), *B. breve* YIT 4014 (Yakult), *B. infantis 35624* (Procter &Gamble).

Les autres souches de bactérie(s) lactique(s) peuvent également être choisies parmi celles qui sont habituellement utilisées pour la préparation de produits alimentaires.

Leur choix dépend du type de produit fermenté que l'on souhaite obtenir :

A titre d'exemples non-limitatifs, on citera :
- des lactobacilles tels que *Lactobacillus acidophilus, Lactobacillus casei, Lactobacillus reuteri, Lactobacillus helveticus, Lactobacillus plantarum,* etc.
- des ferments du yoghourt, à savoir *Streptococcus thermophilus* et *Lactobacillus delbrueckii* subsp. *bulgaricus,* qui peuvent, le cas échéant, être associés à d'autres espèces de *Lactobacillus ;*
- des ferments du kefir, contenant généralement *Lactobacillus kefiri,* associé à des bactéries des genres *Leuconostoc, Lactococcus* et *Acetobacter,* et à des levures des genres *Kluyveromyces* et *Saccharomyces ;*
- des bactéries mésophiles, utilisées notamment pour la fabrication de fromages, tels que *Lactococcus cremoris, Lactococcus lactis*, *Lactococcus diacetylactis* et *Leuconostoc.*

On peut utiliser de la gomme arabique *d'Acacia senegal* ou *d'Acacia seyal,* par exemple celles respectivement commercialisées par la société CNI sous les dénominations FIBREGUM^{®}P et FIBREGUM^{®}B ou celles commercialisées par la société Kerry Ingrédients ou par la société ALFRED L. WOLFF GmbH sous la marque Quick Gum®.

Le (les) acide(s) aminé(s) soufré(s) sera (seront) utilisé(s) de préférence sous forme libre, selon les modalités décrites dans la Demande PCT FR2006/001688, déposée le 11 juillet 2006 au nom de COMPAGNIE GERVAIS DANONE; on peut également utiliser des granules contenant de la cystéine base, similaires à ceux décrits dans la Demande FR 0606421 déposée le 13 juillet 2006 au nom de COMPAGNIE GERVAIS DANONE.

La quantité de gomme arabique dans le produit final (c'est-à-dire le produit fermenté et conditionné) sera généralement d'environ 0,1 à environ 3%, de préférence d'environ 0,2 à environ 1% en poids. La quantité d'acide(s) aminé(s) soufré(s) dans le produit final peut aller d'environ 5 jusqu'à environ 50 mg/l; elle sera généralement d'environ 5 à environ 20 mg/l, de préférence d'environ 5 à environ 15 mg/l, et avantageusement d'environ 5 à environ 10 mg/l.

La quantité de gomme arabique utilisée peut avantageusement être adaptée en fonction du taux protéique visé du produit final, et de l'ajout ou non d'acide(s) aminé(s) soufré(s).

Dans le cas d'un produit dont le taux protéique est supérieur ou égal à 3,4 %, la gomme arabique peut être utilisée seule, de préférence de manière à obtenir une quantité supérieure ou égales à 0,5% dans le produit final ; si l'on souhaite obtenir une quantité de gomme arabique dans le produit final inférieure à 0,5%, il est préférable d'ajouter également le (les) acide(s) aminé(s) soufré(s).

Dans le cas d'un produit dont le taux protéique est inférieur à 3,4 %, il est généralement préférable d'utiliser une combinaison de gomme d'acacia et d'acide(s) aminé(s) soufré(s), notamment pour obtenir un effet optimal sur la survie des bifidobactéries.

Pour obtenir l'effet souhaité sur la viabilité des bifidobactéries, la gomme arabique comme le (les) acide(s) aminé(s) soufré(s) peuvent être ajoutés, ensemble ou séparément, et en une ou plusieurs fois, à tout moment de la fabrication du produit avant la fin de son conditionnement.

Selon un mode de mise en oeuvre préféré du procédé de la présente invention, il comprend une étape de fermentation d'un substrat alimentaire par une ou plusieurs souche(s) de *Bifidobacterium,* en association ou non avec une ou plusieurs souche(s) d'une ou plusieurs bactérie(s) lactique(s) autre(s) que *Bifidobacterium.*

Dans ce cas, afin de stimuler la croissance des Bifidobacterium, l'addition d'au moins une partie de la quantité totale de gomme arabique, et éventuellement d'au moins une partie de la quantité totale de le (les) acide(s) aminé(s) soufré(s), est effectuée préalablement à cette fermentation. Lorsque la quantité de gomme arabique prévue dans le produit final est inférieure à 0,5%, il est préférable que la totalité de la gomme arabique soit ajoutée préalablement à la fermentation ; lorsque la quantité de gomme arabique prévue dans le produit final est de 0,5% à 1%, la quantité de gomme arabique ajoutée préalablement à la fermentation représente de préférence de 50 à 100% de la quantité totale utilisée ; lorsque la quantité de gomme arabique prévue dans le produit final est supérieure à 1 %, la quantité de gomme arabique ajoutée préalablement à la fermentation représente de préférence de 20 à 100% de la quantité totale utilisée. De même, lorsque la quantité d'acide(s) aminé(s) soufré(s) prévue dans le produit final est de 5 à environ 10 mg/l, la quantité d'acide(s) aminé(s) soufré(s) ajoutée préalablement à la fermentation représente de préférence de 80 à 100% de la quantité totale utilisée ; lorsque la quantité d'acide(s) aminé(s) soufré(s) prévue dans le produit final est supérieure à 10 mg/l, la quantité d'acide(s) aminé(s) soufré(s) ajoutée préalablement à la fermentation représente de préférence de 20 à 100% de la quantité totale utilisée.

Selon une modalité particulièrement avantageuse de ce mode de mise en oeuvre, au moins une partie des acides aminés soufrés utilisés est ajoutée lors de l'ensemencement par les bactéries, sous forme de granules contenant de la cystéine base comme décrit dans la Demande FR 0606421, et contenant au moins l'une des souches de *Bifidobacterium* utilisées.

Un procédé conforme à l'invention peut comprendre plusieurs étapes de fermentation, effectuées séparément, mettant en oeuvre des associations différentes de bactéries lactiques, et/ou des substrats alimentaires de nature différente. Ces étapes de fermentation sont généralement effectuées en parallèle, et leurs produits sont ensuite mélangés. Il est possible que l'une de ces fermentations séparées mette en oeuvre un ferment ne comprenant pas de bifidobactéries. Dans ce cas, il n'est pas nécessaire d'effectuer la fermentation en présence de gomme arabique ou de la combinaison gomme arabique/acide(s) aminé(s) soufré(s); il est toutefois possible d'ajouter, ensemble ou séparément, la gomme arabique comme le (les) acide(s) aminé(s) soufré(s), avant, pendant ou après la fermentation, afin de les retrouver dans le mélange des produits de fermentation.

Dans le cadre du procédé de l'invention, la quantité de bifidobactéries utilisée pour l'ensemencement du substrat alimentaire est d'environ 10⁶ à environ 2.10⁷ UFC par ml de substrat. Lorsque les bifidobactéries sont mises en oeuvre pour fermenter un substrat conjointement avec d'autres bactéries lactiques, elles représentent généralement d'environ 20 à environ 75 %, de préférence d'environ 30 à environ 50 %, et de manière particulièrement préférée d'environ 35 à environ 40 %, de la population bactérienne présente dans l'inoculum utilisé.

La quantité de bifidobactéries dans le produit final, immédiatement après fermentation et conditionnement (c'est-à dire dans les 4 heures qui suivent la fin de la fermentation), est d'au moins 1.10⁸, de préférence de l'ordre de 2.10⁸ à 10⁹ UFC par ml de produit.

Lorsque des bifidobactéries sont ajoutées à un produit fermenté par d'autres bactéries lactiques, la quantité ajoutée est également ajustée de manière à obtenir également une population d'au moins 1.10⁸, de préférence de l'ordre de 2.10⁸ à 10⁹ UFC par ml de produit.

Le procédé conforme à l'invention est utilisable pour la fabrication d'une grande variété de produits frais fermentés, et peut être mis en oeuvre sans modification, ou en n'effectuant que des modifications mineures, des modalités usuelles de préparation de ce type de produits.

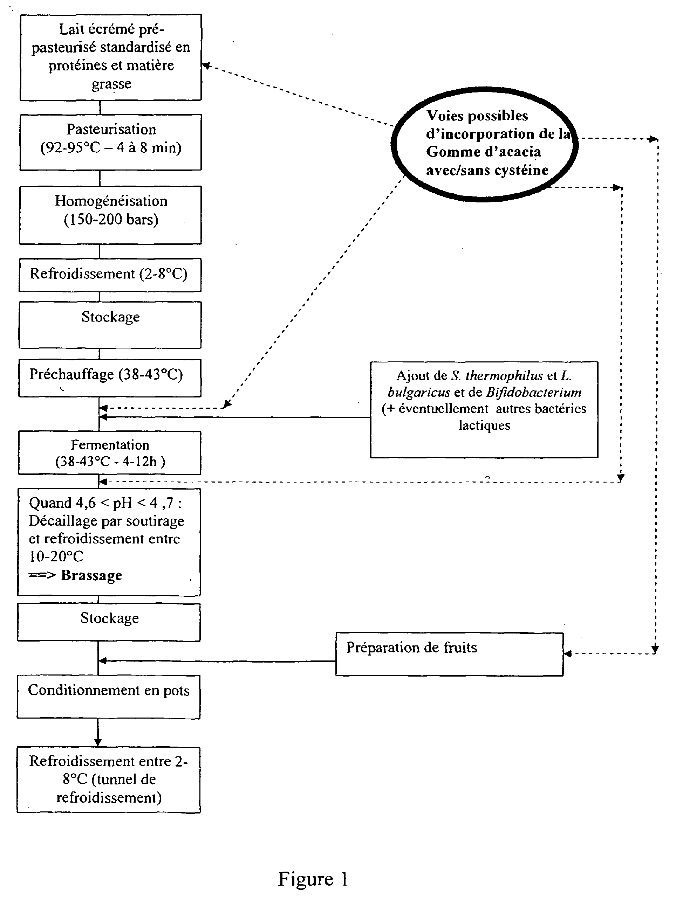

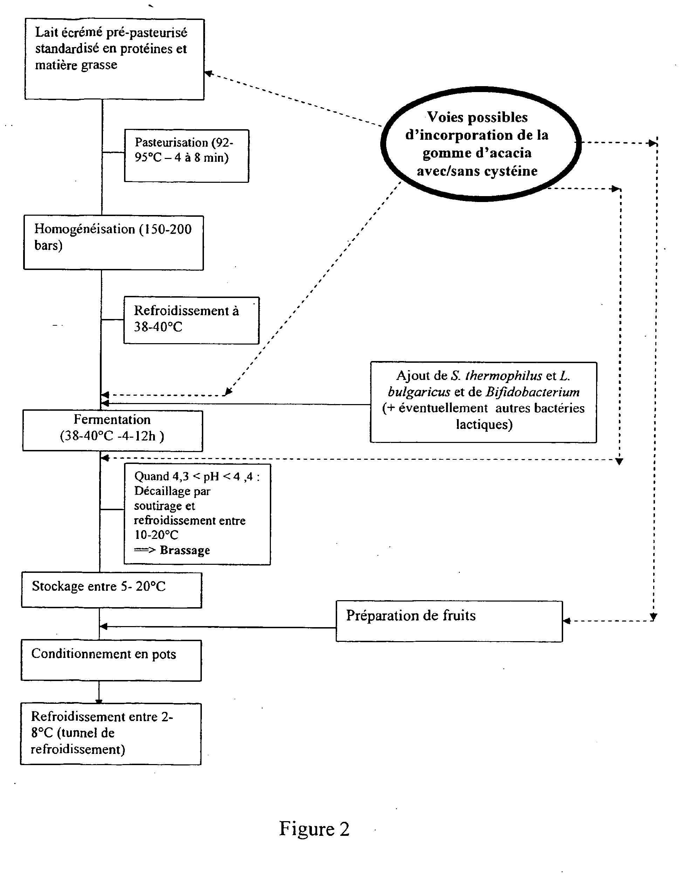

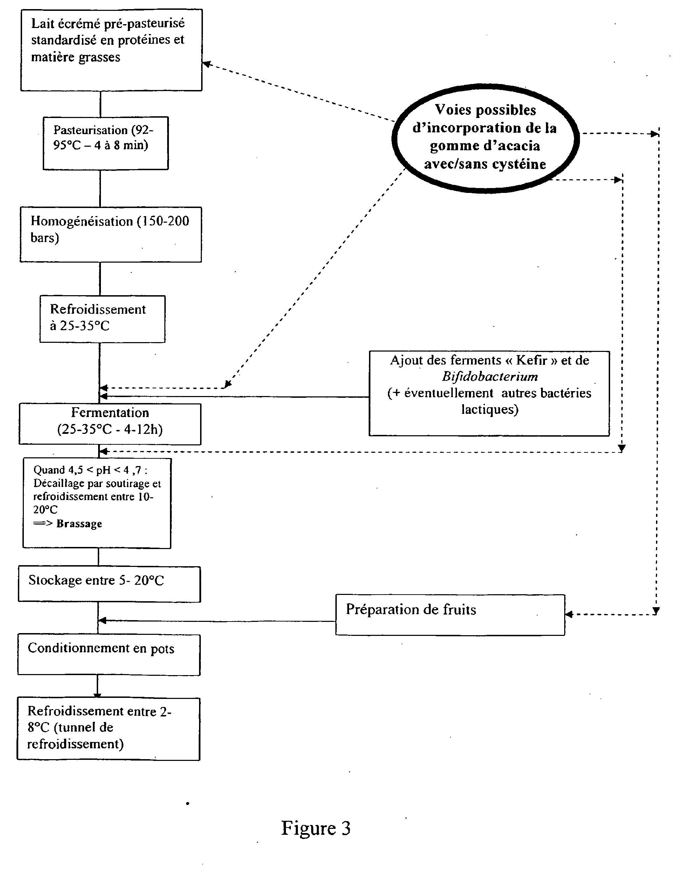

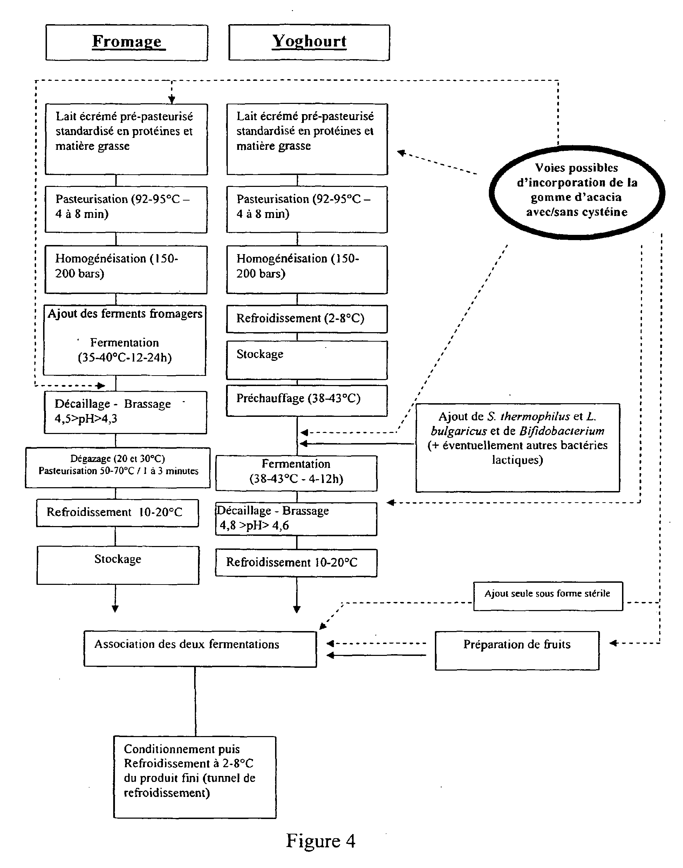

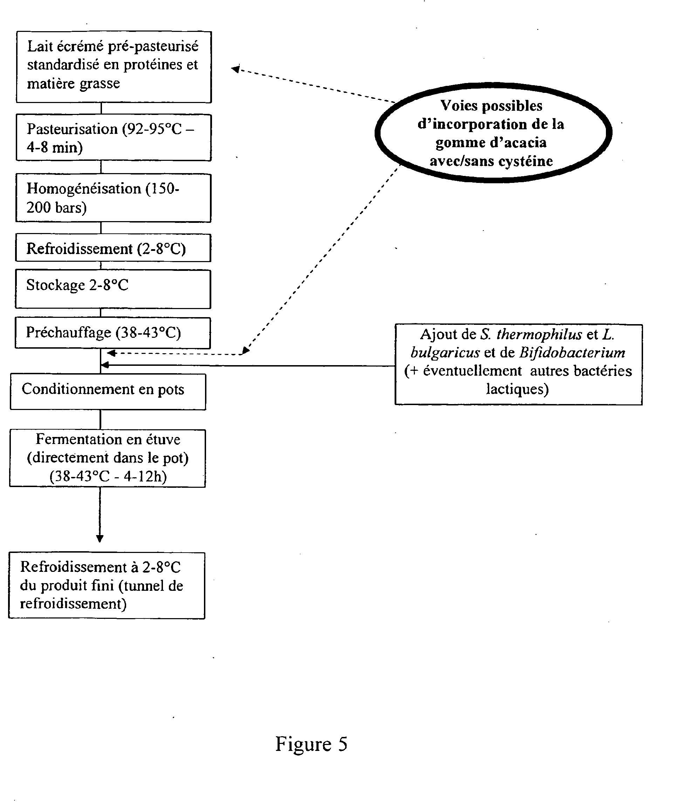

Les Figures 1 à 5 schématisent, à titre d'exemples non-limitatifs, la mise en oeuvre du procédé de l'invention dans le cadre de la préparation de divers produits laitiers fermentés.
La Figure 1 schématise la préparation d'un produit fermenté de type yoghourt brassé ;
La Figure 2 schématise la préparation d'un produit fermenté de type yoghourt à boire ;
La Figure 3 schématise la préparation d'un produit fermenté contenant des ferments du yoghourt et des ferments du kefir ;
La Figure 4 schématise la préparation d'un produit fermenté contenant des ferments du yoghourt et des ferments utilisés en fabrication fromagère ;
La Figure 5 schématise la préparation d'un produit fermenté de type yoghourt ferme.

La présente invention a également pour objet des produits alimentaires fermentés, et notamment des produits laitiers fermentés et/ou à base de substrat végétal, susceptibles d'être obtenus par le procédé conforme à l'invention.

La présente invention a également pour objet des produits alimentaires fermentés se présentant sous la forme de fourrages gras pouvant entrer dans la composition de barres céréalières et de biscuits fourrés.

Des produits alimentaires fermentés conformes à l'invention, contiennent de 0,1 à 3%, de préférence de 0,2 à 1% en poids de gomme arabique, et avantageusement, contiennent également d'environ 5 à environ 50 mg/l d'acide(s) aminé(s) soufré(s), de préférence de cystéine.

Ils contiennent également, immédiatement après fermentation et conditionnement, au moins 1.10⁸, de préférence de l'ordre de 2.10⁸ à 10⁹ UFC de bifidobactéries par ml de produit. De préférence, après 28 jours de conservation entre 4 et 10°C, au moins 40% des bifidobactéries, présentes à l'issue de la fermentation sont encore vivantes dans lesdits produits.

Des produits particulièrement préférés contiennent de 0,2 à 1% en poids de gomme arabique, et de 5 à 10 mg/l de cystéine, après 35 jours de conservation entre 4 et 10°C, au moins 30% des bifidobactéries présentes à l'issue de la fermentation sont encore vivantes dans lesdits produits. De manière particulièrement préférée, il s'agit de produit laitiers fermentés, de type yaourt à boire, contenant de 2 à 3,5% de protéines.

La présente invention sera mieux comprise à l'aide du complément de description qui va suivre, qui se réfère à des exemples non-limitatifs illustrant la mise en oeuvre du procédé conforme à l'invention pour stimuler la croissance et augmenter la survie de bifidobactéries cultivées en présence de ferments du yoghourt.

### EXEMPLE 1 : EFFET DE LA GOMME D'ACACIA SUR LA CROISSANCE ET LA SURVIE DES BIFIDOBACTÉRIES DANS UN PRODUIT LAITIER

### Protocole expérimental

La formule choisie pour l'expérimentation est un lait avec un taux protéique de 4.4% et un taux de 3,5% de matières grasses, obtenu par réhydratation de poudre de lait. C'est une formule donnant normalement une population en bifidobactéries faible.

Deux doses de gomme d'acacia (FIBREGUM^{®}B)ont été testées : 0,5% et 1% en poids dans le produit fini. La gomme d'acacia est incorporée lors de la réhydratation. Le temps de réhydratation est d'une heure.

Le lait additionné de gomme d'acacia a été traité thermiquement selon le protocole suivant : pré-chauffage à 55°C,
pasteurisation à 95°C pendant 6 minutes,
pré-refroidissement à 40°C, refroidissement à 4°C.

Le lait est alors stocké à 4°C jusqu'à son utilisation (dans les 24 heures qui suivent).

### Préparation pour inoculation:

40 minutes avant inoculation, le lait est tempéré à 38°C.

Pour l'ensemencement, l'inoculum suivant est utilisé :
*Streptococcus thermophilus* + *Lactobacillus delbrueckii* ssp. *bulgaricus*+ *Bifidobacterium animalis* ssp *lactis* (CNCM : 1-2494), à raison de 5.10⁶ UFC/ml de *Streptococcus thermophilus,* de 5.10⁶ UFC/ml de *Lactobacillus bulgaricus* et de 1.10⁷ UFC/ml de *Bifidobacterium animalis.*

Après l'inoculation, le lait est replongé dans le bain-marie à 38°C. La fermentation est suivi au CINAC^{®} (YSEBAERT) jusqu'au pH de décaillage (4,8).

### Décaillage, lissage, conditionnement :

A pH=4,8, le lait fermenté est lissé et refroidi à 20°C (soit 6 litres/ h) avec un filtre à mailles 500 µm. Il est ensuite conditionné en pots de 125 ml, puis mis au tunnel froid (4°C) pendant une nuit. Le lendemain matin, les pots sont transférés à 10°C et le suivi microbiologique est effectué jusqu'à J28. Les dénombrements sont réalisés en triple (3 mesures par essai) suivant la méthode à la dicloxacilline décrite par Grand et al., 2003.

### Résultats

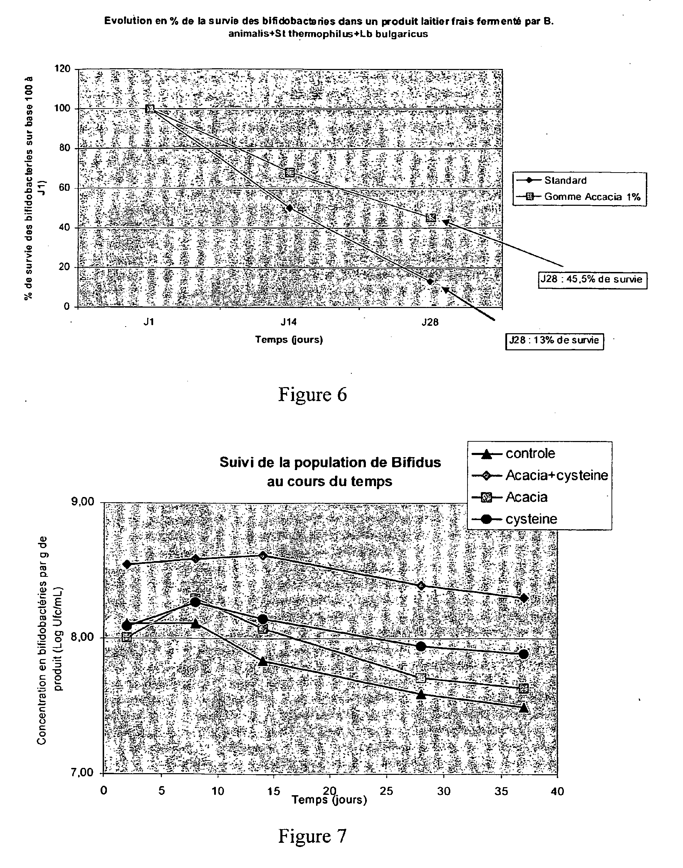

Les résultats sont illustrés par le Tableau I, qui indique les populations de *Bifidobacterium animalis* à J1, J14, et J28, par le Tableau II, qui résume les pourcentages de survie à J14 et J28, et par la Figure 6 qui représente l'évolution en % de la survie des bifidobacteries en fonction du temps.

**Tableau I**

| | pop J1 UFC/ml | pop J14 UFC/ml | pop J28 UFC/ml |
|---|---|---|---|
| Témoin | 2,30E+08 | 1,15E+08 | 3.00E+07 |
| Acacia 1% | 2,20E+08 | 1,50E+08 | 1.20E+08 |

**Tableau II**

| | Fin fermentation | D+14 | D+28 |
|---|---|---|---|
| | J1 | J14 | J28 |
| Témoin | 100 | 50 | 13 |
| Gomme Acacia 1% | 100 | 68 | 45,5 |

Ces résultats montrent nettement une amélioration de la survie de la population bactérienne à J+28 en présence de 1% de gomme d'acacia (les populations à J+1 de l'essai Témoin et de l'essai Acacia étant statistiquement non différentes).

A 0,5%, de gomme d'acacia l'effet est plus faible (résultats non illustrés), mais reste significatif.

### EXEMPLE 2 : EFFET DE L'ASSOCIATION DE LA GOMME D'ACACIA ET DE LA CYSTÉINE SUR LA CROISSANCE ET LA SURVIE DE BIFIDOBACTÉRIES DANS UN PRODUIT LAITIER

### Protocole expérimental

Un yaourt à boire (2,8% de protéines) est fabriqué par réhydratation de poudre de lait, et additionné de 0,8% (en poids) de gomme d'acacia, comme décrit à l'Exemple 1 ci-dessus. Ce type de produit a été choisi pour les essais, dans la mesure où c'est celui qui pose le plus de problèmes en termes de maintien de population de bifidobactéries au cours du stockage. Le lait réhydraté additionné de gomme d'acacia est traité thermiquement comme décrit à l'Exemple 1 ci-dessus, et homogénéisé (150-200 bars).

Après refroidissement à 38-40°C, *Streptococcus thermophilus* et *Lactobacillus delbrueckii* ssp. *bulgaricus* sont ajoutés à raison de 5.10⁶ UFC/ml de *Streptococcus thermophilus,* de 5.10⁶ UFC/ml de *Lactobacillus bulgaricus. Bifidobacterium animalis* ssp *lactis* 1-2494 est ajouté sous la forme de ferment à ensemencement direct, tels que ceux décrits dans la Demande FR 0606421 supplémenté en cystéine ou non. Le ferment supplémenté utilisé correspond à l'ajout de 1,92. 10⁷ UFC/ml de *Bifidobacterium animalis* et 6,9 mg/l de cystéine.

Après l'inoculation, la fermentation est effectuée à 38°C, et suivie au CINAC® (YSEBAERT) jusqu'au pH de décaillage (4,3 < pH < 4,4).

Le décaillage est effectué par soutirage et refroidissement entre 10-20°C, puis brassage.

Le produit est ensuite conditionné en pots puis mis au tunnel froid (4°C).

Des comptages de populations sont ensuite réalisés à 2h de stockage à 15°C puis 2, 8, 14, 28 et 37 jours de stockage à 10°C.

### Résultats

Les résultats sont illustrés par le Tableau III ci-dessous, et par la Figure 7, qui représente le suivi de la population de bifidobactéries en fonction du temps.

Ces résultats montrent un très net effet de la gomme d'acacia, et de l'association gomme d'acacia + cystéine sur la croissance des bifidobactéries. Dans le cas de l'association gomme d'acacia + cystéine on observe en outre un effet particulièrement net sur la survie des bifidobactéries au cours de la conservation.

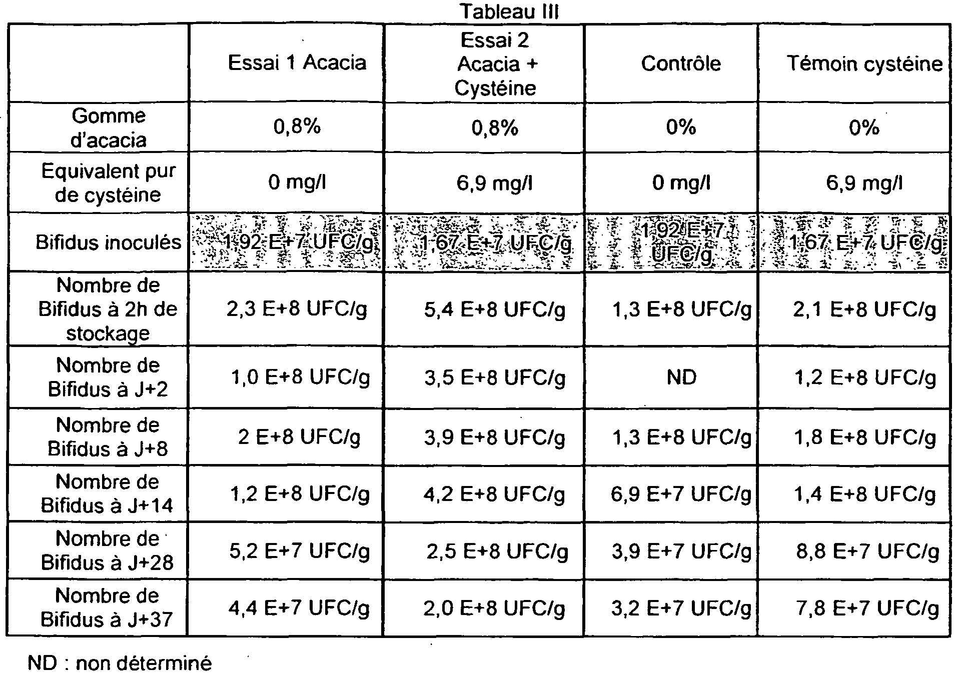

La même expérimentation a été reproduite avec un produit à 2,7% de protéines, contenant 0,8% de gomme d'acacia, et ensemencé avec un ferment supplémenté en cystéine, correspondant à l'ajout de 1,90. 10⁷ UFC/ml de *Bifidobacterium animalis* et 7 mg/l de cystéine.

Les résultats sont illustrés par le Tableau IV.

Ces résultats confirment l'effet de la gomme d'acacia, et surtout de l'association gomme d'acacia + cystéine sur la survie des bifidobactéries. 52 jours après inoculation, les bifidobactéries survivantes sont en effet 2 fois plus nombreuses dans le produit additionné de gomme d'acacia, et 10 fois plus nombreuses dans le produit additionné de gomme d'acacia et de cystéine que dans le produit contrôle.

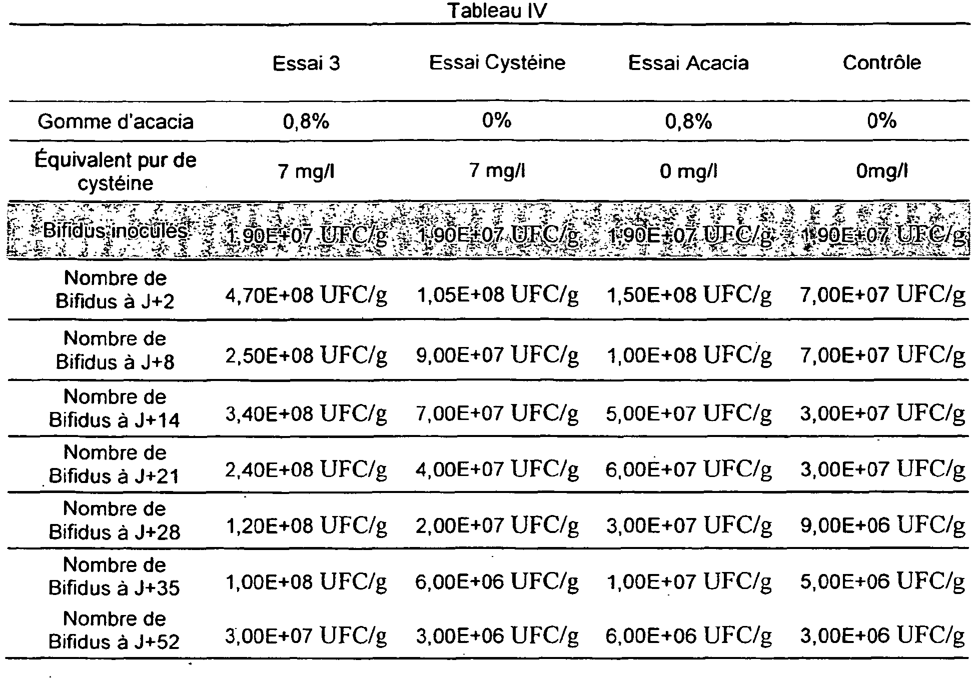

### Références bibliographiques

- Lian et al. ; Survival of Bifidobacteria after spray-drying. International Journal of Food Microbiology, 2002, vol. 74, N°1/2, p. 79-86.
- Lian et al. ; Viability of microencapsulated bifidobacteria in simulated gastric juice and bile solution. International Journal of Food Microbiology, 2003 vol. 86, n°3, p. 293-301.
- Hsiao et al. 2004 ; Effect of packaging conditions and temperature on viability of microencapsulated bifidobacteria during storage. Journal of Science of Food and Agriculture, 2004, vol. 84, n°2, p. 134-139.
- Desmond et al. 2002. Improved survival of Lactobacillus paracasei NFBC 338 in spray-dried powders containing gum acacia. Journal of Applied Microbiology, 2002, vol. 93, p. 1003-1011.
- Wyatt et al. 1986 ; A change in human faecal flora in response to inclusion of gum arabic in the diet. British Journal ofNutrition, 1986, vol.55, n°2, p. 261-266.
- Cherbut et al. 2003 ; Acacia gum is a bifidogenic dietary fibre with high digestive tolerance in healthy humans. Microbial Ecology in Health and Disease, 2003, vol. 15, n°1, p. 43-50.
- Grand et al., 2003 ; Quantitative analysis and molecular identification of bifidobacteria strains in probiotic milk products. European Food Research and Technology, vol. 217, p. 90-92.

## Revendications

1. Procédé pour augmenter la viabilité des bifidobactéries au cours de la conservation d'un produit alimentaire frais fermenté contenant une ou plusieurs souche(s) de *Bifidobacterium* **caractérisé en ce qu'**il comprend l'addition de gomme arabique audit produit alimentaire préalablement à la fin de son conditionnement.

2. Procédé selon la revendication 1, **caractérisé en ce que** ledit produit alimentaire contient en outre une ou plusieurs souche(s) de bactérie(s) lactique(s) autres que *Bifidobacterium.*

3. Procédé selon une quelconque des revendications 1 ou 2, **caractérisé en ce qu'**il comprend en outre l'addition d'au moins un acide aminé soufré audit produit alimentaire préalablement à la fin de son conditionnement.

4. Procédé selon la revendication 3, **caractérisé en ce que** ledit acide aminé soufré est de la cystéine.

5. Procédé selon une quelconque des revendications 1 à 4, **caractérisé en ce que** la gomme arabique est ajoutée de manière à ce que sa concentration finale dans le produit alimentaire soit d'environ 0,1 à environ 3% en poids.

6. Procédé selon une quelconque des revendications 3 à 5, **caractérisé en ce que** le (les) acide(s) aminé(s) soufré(s) est (sont) ajouté(s) de manière à ce que sa (leur) concentration finale soit d'environ 5 jusqu'à environ 50 mg/l.

7. Procédé selon une quelconque des revendications 1 à 6, **caractérisé en ce qu'**il comprend une étape de fermentation d'un substrat alimentaire approprié par un ferment comprenant une ou plusieurs souche(s) de *Bifidobacterium* éventuellement associée(s) avec au moins une souche d'une bactérie lactique autre que *Bifidobacterium,* et **en ce que** afin de stimuler la croissance desdites *Bifidobacterium,* l'addition d'au moins une partie de la quantité totale de gomme arabique, et éventuellement d'au moins une partie de la quantité totale d'acide aminé soufré est effectuée préalablement à ladite étape de fermentation.

8. Procédé selon une quelconque des revendications 1 à 7, **caractérisé en ce que** ledit produit fermenté contient au moins une souche de *Bifidobacterium animalis.*

9. Procédé selon une quelconque des revendications 1 à 8, **caractérisé en ce que** les bactéries lactiques autres que *Bifidobacterium* comprennent une ou plusieurs souches de *Streptococcus thermophilus* et une ou plusieurs souches de *Lactobacillus delbrueckii* subsp. *bulgaricus.*

10. Produit alimentaire fermenté susceptible d'être obtenu par un procédé selon une quelconque des revendications 1 à 9.

11. Produit alimentaire fermenté selon la revendication 10, **caractérisé en ce qu'**il s'agit d'un produit laitier fermenté.

12. Produit alimentaire fermenté selon la revendication 10, **caractérisé en ce qu'**il s'agit d'un produit à base de substrat végétal fermenté.

13. Produit alimentaire fermenté selon la revendication 10, **caractérisé en ce qu'**il s'agit d'un fourrage gras.

## Patentansprüche

1. Verfahren zum Steigern der Lebensfähigkeit von Bifidobakterien während der Konservierung eines fermentierten frischen Nahrungsmittelprodukts, das ein oder mehrere Stämme von *Bifidobacterium* enthält, **dadurch gekennzeichnet, dass** es die Zugabe von Gummi arabicum zu dem Nahrungsmittelprodukt vor dem Ende seiner Verpackung umfasst.

2. Verfahren nach Anspruch 1, **dadurch gekennzeichnet, dass** das Nahrungsmittelprodukt weiterhin ein oder mehrere andere Stämme von Milchsäurebakterien als *Bifidobacterium* enthält.

3. Verfahren nach einem der Ansprüche 1 oder 2, **dadurch gekennzeichnet, dass** es weiterhin die Zugabe wenigstens einer schwefelhaltigen Aminosäure zu dem Nahrungsmittelprodukt vor dem Ende seiner Verpackung umfasst.

4. Verfahren nach Anspruch 3, **dadurch gekennzeichnet, dass** die schwefelhaltige Aminosäure Cystein ist.

5. Verfahren nach einem der Ansprüche 1 bis 4, **dadurch gekennzeichnet, dass** der Gummi arabicum so zugegeben wird, dass seine Endkonzentration in dem Nahrungsmittelprodukt etwa 0,1 bis etwa 3 Gew.-% beträgt.

6. Verfahren nach einem der Ansprüche 3 bis 5, **dadurch gekennzeichnet, dass** die schwefelhaltige(n) Aminosäure(n) so zugegeben wird (werden), dass ihre Endkonzentration etwa 5 bis etwa 50 mg/l beträgt.

7. Verfahren nach einem der Ansprüche 1 bis 6, **dadurch gekennzeichnet, dass** es einen Schritt des Fermentierens eines geeigneten Nahrungsmittelsubstrats durch ein Gärmittel umfasst, das ein oder mehrere Stämme von *Bifidobacterium* gegebenenfalls in Kombination mit wenigstens einem anderen Stamm eines Milchsäurebakteriums als *Bifidobacterium* umfasst, und dadurch, dass die Zugabe wenigstens eines Teils der Gesamtmenge von Gummi arabicum und gegebenenfalls wenigstens eines Teils der Gesamtmenge schwefelhaltiger Aminosäure zur Wachstumsstimulierung der *Bifidobacterium* vor dem Fermentierungsschritt erfolgt.

8. Verfahren nach einem der Ansprüche 1 bis 7, **dadurch gekennzeichnet, dass** das fermentierte Produkt wenigstens einen Stamm von *Bifidobacterium animalis* enthält.

9. Verfahren nach einem der Ansprüche 1 bis 8, **dadurch gekennzeichnet, dass** die anderen Milchsäurebakterien als *Bifidobacterium* ein oder mehrere Stämme von *Streptococcus thermophilus* und ein oder mehrere Stämme von *Lactobacillus delbrueckii* subsp. *bulgaricus* umfassen.

10. Fermentiertes Nahrungsmittelprodukt, erhältlich durch ein Verfahren nach einem der Ansprüche 1 bis 9.

11. Fermentiertes Nahrungsmittelprodukt nach Anspruch 10. **dadurch gekennzeichnet, dass** es ein fermentiertes Milchprodukt ist.

12. Fermentiertes Nahrungsmittelprodukt nach Anspruch 10, **dadurch gekennzeichnet, dass** es ein Produkt auf Basis eines fermentierten pflanzlichen Substrats ist.

13. Fermentiertes Nahrungsmittelprodukt nach Anspruch 10, **dadurch gekennzeichnet, dass** es eine Fettfüllung ist.

## Claims

1. Method of increasing the viability of bifidobacteria during the preservation of a fermented fresh food product containing one or more strains of *Bifidobacterium*, **characterised in that** it comprises the addition of gum arabic to said food product before the end of its packaging.

2. Method as claimed in claim 1, **characterised in that** said food product additionally contains one or more strains of lactic acid bacteria other than *Bifidobacterium.*

3. Method as claimed in any one of claims 1 or 2, **characterised in that** it further comprises the addition of at least one sulphur-containing amino acid to said food product before the end of its packaging.

4. Method as claimed in claim 3, **characterised in that** said sulphur-containing acid is cysteine.

5. Method as claimed in any one of claims 1 to 4, **characterised in that** the gum arabic is added so that its final concentration in the food product is approximately 0.1 to approximately 3% by weight.

6. Method as claimed in any one of claims 3 to 5, **characterised in that** the sulphur-containing amino acid(s) is (are) added so that its (their) final concentration is approximately 5 to approximately 50 mg/l.

7. Method as claimed in any one of claims 1 to 6, **characterised in that** it comprises a step of fermenting an appropriate food substrate by a ferment comprising one or more strains *of Bifidobacterium* optionally in conjunction with at least one strain of a lactic acid bacterium other than *Bifidobacterium*, and in order to stimulate growth of said *Bifidobacterium*, at least some of the total quantity of gum arabic and optionally at least some of the total quantity of sulphur-containing amino acid is added prior to said fermentation step.

8. Method as claimed in any one of claims 1 to 7, **characterised in that** said fermented product contains at least one strain of *Bifidobacterium animalis.*

9. Method as claimed in any one of claims 1 to 8, **characterised in that** the lactic acid bacteria other than *Bifidobacterium* comprise one or more strains of *Streptococcus thermophilus* and one or more strains of *Lactobacillus delbrueckii* subsp. *bulgaricus.*

10. Fermented food product which can be obtained by a method as claimed in any one of claims 1 to 9.

11. Fermented food product as claimed in claim 10, **characterised in that** it is a fermented dairy product.

12. Fermented food product as claimed in claim 10. **characterised in that** it is a product with a fermented plant substrate.

13. Fermented food product as claimed in claim 10, **characterised in that** it is a fat-based tilling.
